# EUROPEAN PATENT APPLICATION

(11) **EP 4 245 282 A1**
(43) Date of publication of application: **20.09.2023**
(21) Application number: 20961683.8
(22) Date of filing: 12.11.2020
(51) Int. Cl.: A61H 23/00, A61N 1/36, A61N 1/32, A61H 23/02, A61N 1/04

(54) **EXTRACORPOREAL SHOCK WAVE THERAPY APPARATUS HAVING ADDED HIGH-FREQUENCY AND LOW-FREQUENCY TREATMENT FUNCTIONS**

(71) Applicant: ITC Co., Ltd, Daejeon 34036 (KR)
(72) Inventor: LEE, Keun Deok, Daejeon 34036 (KR)
(74) Representative: M. Zardi & Co S.A.
(86) International application number: PCT/KR2020/015901
(87) International publication number: WO 2022/102813

(57) **Abstract**

Provided is an extracorporeal shock wave medical device with high-frequency and low-frequency treatment functions. The present invention includes a body portion, a cylinder disposed inside the body portion, a moving portion which linearly moves inside the cylinder, a connecting portion disposed at the end of the cylinder and connected to the body portion to selectively collide with the moving portion when the moving portion moves, an impact portion connected to the connecting portion and configured to transmit an impact to the outside when the moving portion collides with the connecting portion, and a waveform transmitting portion electrically connected to the impact portion to transmit a high frequency or low frequency to the impact portion.

## Description

### Technical Field

Embodiments of the present invention relate to a device, and more particularly, to an extracorporeal shock wave medical device with high-frequency and low-frequency treatment functions.

### Background Art

In general, extracorporeal shock wave medical devices are applicable to various lesions. In this case, the extracorporeal shock wave medical devices may include various functions in order to achieve the maximum effect on various types of lesions. For example, the extracorporeal shock wave medical devices may generate mechanical vibration and apply the vibration to the user's body to stimulate the lesion.

### Technical Problem

However, since the above-described extracorporeal shock wave medical device needs to be applied to various affected areas of a user, it is necessary to have various functions. For example, an extracorporeal shock wave medical device needs to produce endogenous dermal heat in an affected area of the human body by applying a high-frequency electrical stimulation signal to the body in addition to mechanical vibration, or an extracorporeal shock wave medical device needs to apply a low-frequency electrical stimulation signal to the user's body.

In order to solve this problem, it is necessary to develop an extracorporeal shock wave medical device capable of simultaneously implementing two functions.

### Technical Solution

An object of the present invention is to provide an extracorporeal shock wave medical device capable of applying physical shock and electric shock.

One embodiment of the present invention discloses an extracorporeal shock wave medical device with high-frequency and low-frequency treatment functions, the extracorporeal shock wave medical device including: a body portion, a cylinder disposed inside the body portion, a moving portion which linearly moves inside the cylinder, a connecting portion disposed at the end of the cylinder and connected to the body portion to selectively collide with the moving portion when the moving portion moves, an impact portion connected to the connecting portion and configured to transmit an impact to the outside when the moving portion collides with the connecting portion, and a waveform transmitting portion electrically connected to the impact portion to transmit a high frequency or low frequency to the impact portion.

### Advantageous Effects

An extracorporeal shock wave medical device according to the embodiments of the present invention may simultaneously provide electrical stimulation and physical stimulation through simple operation. In addition, the extracorporeal shock wave medical device according to the embodiments of the present invention may minimize the occurrence of excessive electrical stimulation when an electrical signal is turned on/off.

### Description of Drawings

FIG. 1 is a perspective view of an extracorporeal shock wave medical device according to an embodiment of the present invention.
FIG. 2 is an exploded perspective view of the extracorporeal shock wave medical device shown in FIG. 1.
FIG. 3 is a cross-sectional view taken along line III-III' of FIG. 1.
FIG. 4 is a cross-sectional view showing a connecting portion, an impact portion, and a contact holding portion shown in FIG. 2.
FIG. 5 is a cross-sectional view showing a connecting portion, an impact portion, and a contact holding portion of an extracorporeal shock wave medical device according to another embodiment of the present invention.
FIG. 6 is a cross-sectional view showing a connecting portion, an impact portion, and a contact holding portion of an extracorporeal shock wave medical device according to still another embodiment of the present invention.
FIG. 7 is a cross-sectional view showing a connecting portion, an impact portion, and a contact holding portion of an extracorporeal shock wave medical device according to another embodiment of the present invention.

### Best Mode

One embodiment of the present invention discloses an extracorporeal shock wave medical device with high-frequency and low-frequency treatment functions, the extracorporeal shock wave medical device including: a body portion, a cylinder disposed inside the body portion, a moving portion which linearly moves inside the cylinder, a connecting portion disposed at the end of the cylinder and connected to the body portion to selectively collide with the moving portion when the moving portion moves, an impact portion connected to the connecting portion and configured to transmit an impact to the outside when the moving portion collides with the connecting portion, and a waveform transmitting portion electrically connected to the impact portion to transmit a high frequency or low frequency to the impact portion.

In the present embodiment, the extracorporeal shock wave medical device may further include a cap portion connected to the impact portion to transmit the high frequency or low frequency to a user.

In the present embodiment, the extracorporeal shock wave medical device may further include an outer case portion disposed to surround the outside of the body portion.

In the present embodiment, the extracorporeal shock wave medical device may further include a contact holding portion disposed between the connecting portion and the impact portion to electrically connect the connecting portion and the impact portion.

In the present embodiment, the contact holding portion may include a spring which is a conductive material.

In the present embodiment, the connecting portion may include a connecting body portion into which a part of the impact portion is inserted, and a protruding portion which protrudes to the inside of the connecting body portion and is in contact with a part of the impact portion.

Additional aspects, features, and advantages, other than those described above, will be apparent from the drawings, claims, and written description below.

These general and specific aspects may be embodied using systems, methods, computer programs, or any combinations thereof.

### Mode for Invention

As the present invention allows for various changes and numerous embodiments, particular embodiments will be illustrated in drawings and described in detail in the written description. Advantageous effects, features, and methods for achieving the effects and features will become more apparent by explaining the embodiments in detail with reference to the accompanying drawings. However, the present disclosure is not limited to these embodiments but may be realized in various modes.

The embodiments of the present disclosure will now be described more fully with reference to the accompanying drawings. Like reference numerals in the drawings denote like elements, and thus their description will be omitted.

The terms first, second, etc. are used in the description of the embodiments to only distinguish one element from another.

As used herein, the singular forms "a," "an," and "the" are intended to include the plural forms as well, unless the context clearly indicates otherwise.

It will be further understood that the terms "comprises" and/or "comprising," or "includes" and/or "including," when used in this specification, specify the presence of stated features or elements, but do not preclude the presence or addition of one or more other features or elements.

In the drawings, the size of elements may be exaggerated or reduced for clarity. For instance, the size and shape of each element may be arbitrarily illustrated in the drawings, and therefore, the present disclosure is not limited to the drawings.

In the following embodiments, an x-axis, a y-axis, and a z-axis are not limited to three axes on the Cartesian coordinate system, and may be interpreted in a broad sense including the three axes. For example, the x-axis, the y-axis, and the z-axis may be orthogonal to each other, but may refer to different directions that are not orthogonal to each other.

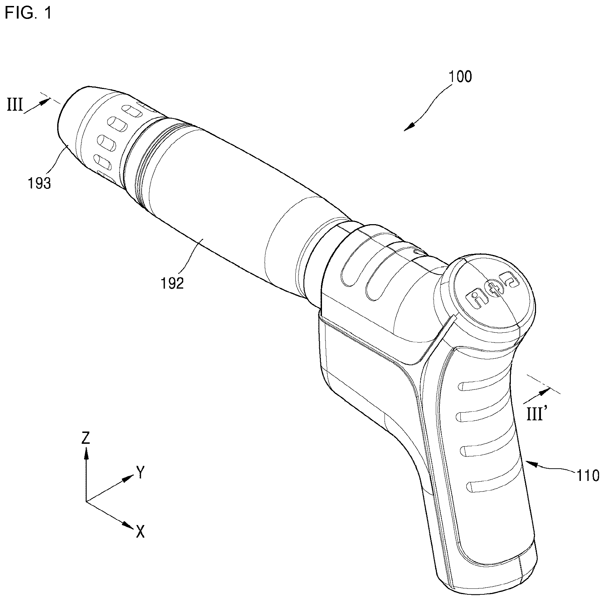

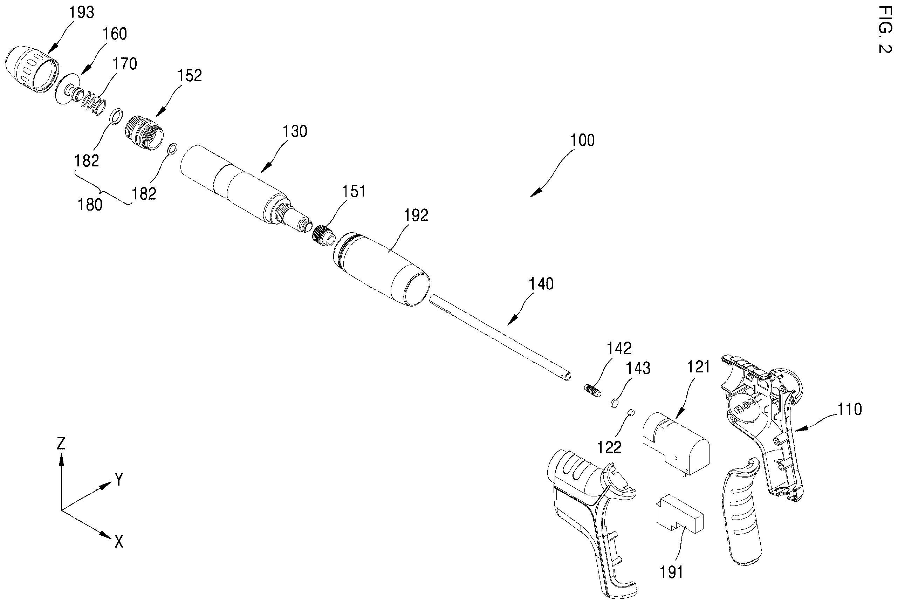

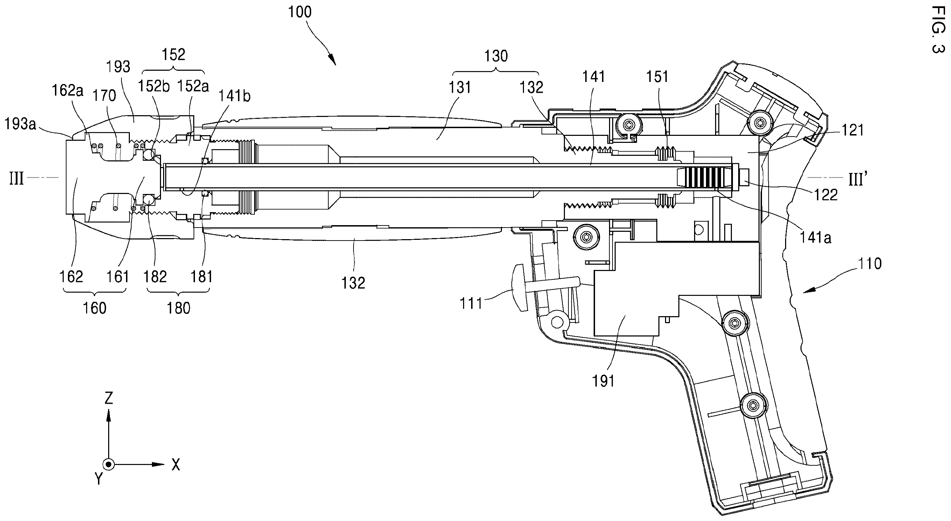

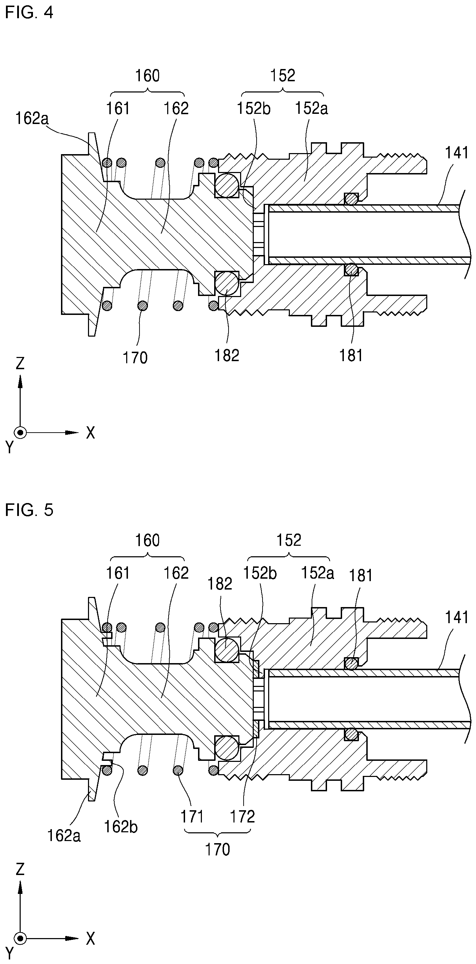

FIG. 1 is a perspective view of an extracorporeal shock wave medical device with high-frequency and low-frequency treatment functions according to an embodiment of the present invention. FIG. 2 is an exploded perspective view of the extracorporeal shock wave medical device shown in FIG. 1. FIG. 3 is a cross-sectional view taken along line III-III' of FIG. 1. FIG. 4 is a cross-sectional view showing a connecting portion, an impact portion, and a contact holding portion shown in FIG. 2.

Referring to FIGS. 1 to 4, an extracorporeal shock wave medical device 100 with high-frequency and low-frequency treatment functions (hereinafter, "extracorporeal shock wave medical device") may include a handle portion 110, a core portion 121, and a magnet portion 122, a body portion 130, a cylinder 141, a moving portion 142, a first connecting portion 151, a second connecting portion 152, an impact portion 160, a waveform transmitting portion 191, a contact holding portion 170, a sealing portion 180, an outer case portion 192, and a cap portion 193.

The handle portion 110 may be gripped by a user. In this case, the handle portion 110 may have a space formed therein, and may be formed of an insulating material. Various devices may be disposed inside the handle portion 110. For example, a pump connected to the cylinder 141 to supply a gas may be disposed in the handle portion 110. The pump is not limited to the above example, such that the pump may be disposed outside the handle portion 110 and connected to the cylinder 141 through a pipe or the like. A controller (not shown) may be included inside the handle portion 110. The waveform transmitting portion 191 may also be included inside the handle portion 110. A battery electrically connected to various devices may be disposed inside the handle portion 110.

Embodiments of the present invention are not limited to those described above, such that the pump, the controller, the waveform transmitting portion 191, the battery, and the like may be disposed outside the handle portion 110. Hereinafter, for convenience of description, a detailed description will be given of an example in which the pump, the controller, the waveform transmitting portion 191, the battery, and the like are disposed outside the handle portion 110.

The core portion 121 may be disposed inside the handle portion 110. In this case, the core portion 121 may be fixed to the handle portion 110 via screws, bolts, or the like. The core portion 121 may be electrically connected to the waveform transmitting portion 191 to transmit a high-frequency or low-frequency signal sent from the waveform transmitting portion 191. In addition, the core portion 121 may be connected to a pump and may form a passage through which a gas moves.

The magnet portion 122 may be disposed in the core portion 121. In this case, the magnet portion 122 may allow the moving portion 142 to be fixed at a position or disposed at a set position by supplying a magnetic or electromagnetic force to the moving portion 142. The magnet portion 122 as described above may include a permanent magnetic and/or an electromagnet.

The body portion 130 may be connected to the handle portion 110. In this case, the body portion 130 may include a main body 131 having a space inside thereof and a protruding connecting portion 132 protruding from the main body 131. In this case, the protruding connecting portion 132 may be inserted into and coupled to the handle portion 110. In addition, a first connecting portion 151 may be disposed on the protruding connecting portion 132. In this case, the first connecting portion 151 may be connected to the protruding connecting portion 132 through screw coupling or the like. Also, the first connecting portion 151 may be connected to the protruding connecting portion 132 to connect the handle portion 110 and/or the core portion 121 and the body portion 130. In this case, since the first connecting portion 151 includes an insulating material, an electrical signal generated in the body portion 130 may be prevented from being transmitted to the body portion 130. In another embodiment, the first connecting portion 151 may be formed of a metal material.

The cylinder 141 may be disposed inside the body portion 130. In this case, the cylinder 141 has a space formed therein, and the moving portion 142 may linearly move in this space. Also, the cylinder 141 may be connected to the core portion121. In addition, a through-hole 141b through which a gas is discharged or introduced when the moving portion 142 moves may be formed at one end of the cylinder 141. The through-hole 141b may be disposed in vicinity of the cap portion 193. In this case, the through-hole 141b may allow air to flow from the outside of the cylinder 141 to the inside when the moving portion 142 moves backward inside the cylinder 141. An inlet hole 141a through which the gas supplied through the core portion 121 is introduced may be disposed at the other end of the cylinder 141.

The moving portion 142 may be disposed inside the cylinder 141 and may linearly move in the longitudinal direction of the cylinder 141. In this case, the moving portion 142 may move forward or backward inside the cylinder 141 according to the operation of the pump. Also, the moving portion 142 may move backward inside the cylinder 141 by the magnet portion 122.

The moving portion 142 as described above may have a curved outer surface. For example, at least one groove may be formed on the outer surface of the moving portion 142. In this case, the groove on the outer surface of the moving portion 142 may be formed in a spiral or annular shape. In particular, in the above case, the moving portion 142 may reduce the contact area between the moving portion 142 and the cylinder 141 when moving within the cylinder 141, thereby reducing friction therebetween.

The second connecting portion 152 may be connected to the body portion 130. In this case, an end of the cylinder 141 may be disposed inside the second connecting portion 152. In the above case, the outer surface of the second connecting portion 152 may be formed of an insulating material or coated with an insulating material.

The second connecting portion 152 as described above may include a cylindrical second connecting body portion 152a and a second protruding portion 152b protruding into the second connecting body portion 152a. Here, the second protruding portion 152b may be formed in an annular shape and may have a hole inside thereof. In this case, the hole may be formed such that the size thereof is smaller than the size of a cross-section perpendicular to the longitudinal direction of the moving portion 142, and thereby may not allow the moving portion 142 to pass therethrough. In addition, the second protruding portion 152b may be formed of a metal material. In particular, an insulating material may not be disposed on the outer surface of the second protruding portion 152b.

The impact portion 160 may be disposed such that an end thereof is inserted into the second connecting portion 152. In this case, the impact portion 160 may include an inserted portion 161 which is inserted into the second connecting portion 152 and an impact transfer portion 162 extending from the inserted portion 161 and configured to transfer an impact. The impact portion 160 may be formed of a metal material. The impact transfer portion 162 may be formed to have a stepped end. For example, the impact transfer portion 162 may include a protrusion 162a, and the cap portion 193 may include at the end thereof a bent portion 193a bent inwardly of the cap portion 193 to catch the protrusion 162a. Accordingly, when the impact transfer portion 162 vibrates, it is possible to prevent separation between the impact transfer portion 162 and the cap portion 193.

The waveform transmitting portion 191 may be connected to the core portion 121 to transmit high-frequency and/or low-frequency signals to the core portion 121, or may be connected to the cylinder 141 to transmit high-frequency and/or low-frequency signals to the cylinder 141. Hereinafter, for convenience of explanation, a detailed description will be given of an example in which the waveform transmitting portion 191 is electrically connected to the core portion 121.

The waveform transmitting portion 191 may transmit a signal to the core portion 121, the core portion 121 may transmit the signal to the second connecting portion 152 through the cylinder 141, and the second connecting portion 152 may transmit the signal to the impact portion 160. In this case, the waveform transmitting portion 191 may transmit a high-frequency signal and/or a low-frequency signal as an electrical signal.

The contact holding portion 170 may be disposed between the impact portion 160 and the second connecting portion 152 and/or between the second connecting portion 152 and the cylinder 141. Hereinafter, for convenience of explanation, a detailed description will be given of an example in which the contact holding portion 170 is disposed between the impact portion 160 and the second connecting portion 152.

The contact holding portion 170 as described above may include a spring. Here, the contact holding portion 170 may be disposed to surround the second protruding portion 152b. In this case, when the contact holding portion 170 is compressed, the second protruding portion 152b may guide a compression direction of the contact holding portion 170 as well as prevent the contact holding portion 170 from being bent. One end of the contact holding portion 170 may be disposed inside the second connecting portion 152 and the other end of the contact holding portion 170 may be supported by the impact transfer portion 162. In this case, the inner diameter of the contact holding portion 170 may be greater than the outer diameter of the inserted portion 161, and the outer diameter of the contact holding portion 170 may be smaller than the inner diameter of the second connecting portion 152. In addition, the impact transfer portion 162 and/or the second connecting portion 152 that are in contact with the contact holding portion 170 may include a protrusion that protrudes inwardly of the contact holding portion 170 and prevents the end of the contact holding portion 170 from moving. In another embodiment, the impact transfer portion 162 and/or the second connecting portion 152 that are in contact with the contact holding portion 170 may have a groove into which the end of the contact holding portion 170 is inserted and seated. In still another embodiment, a magnet may be disposed on the end of the contact holding portion 170 to fix the contact holding portion 170 to the impact transfer portion 162 and/or the second connecting portion 152.

The contact holding portion 170 may be in contact with each of the second connecting portion 152 and the impact portion 160, thereby electrically connecting the second connecting portion 152 and the impact portion 160. Also, the contact holding portion 170 may prevent the second connecting portion 152 and the cylinder 141 from being spaced apart from each other. In particular, when the moving portion 142 hits the second connecting portion 152, it is possible to prevent the second connecting portion 152 and the cylinder 141 from separating apart from each other or prevent the second connecting portion 152 and the impact portion 160 from separating apart from each other.

The sealing portion 180 may be disposed between various components to prevent gas from moving to the outside or from moving to the inside. In this case, the sealing portion 180 may be formed of an elastic material, such as rubber, silicone, or the like. The sealing portion 180 may include a first sealing portion 181 interposed between the cylinder 141 and the second connecting portion 152 and a second sealing portion 182 interposed between the second connecting portion 152 and the impact portion 160. Also, a groove into which the first sealing portion 181 is inserted and seated may be formed on the inner surface of the cylinder 141 and/or the inner surface of the second connecting portion 152. A groove into which the second sealing portion 182 is inserted and seated may be formed on the inner surface of the second connecting portion 152 and/or the outer surface of the impact portion 160.

The first sealing portion 181 and the second sealing portion 182 as described above may couple other components. In this case, the first sealing portion 181 and the second sealing portion 182 may not only allow a certain degree of play between the components coupled to each other, but may also reduce transmission of the vibration, which is applied during the movement of the moving portion 142, in the longitudinal direction and the vertical direction of the cylinder 141. In addition, the first sealing portion 181 and the second sealing portion 182 may reduce the time required for assembling each component during the manufacture of the extracorporeal shock wave medical device 100.

The outer case portion 192 may be disposed to surround the outer surface of the body portion 130 and coupled to the handle portion 110. Also, the outer case portion 192 may be coupled to the cap portion 193. In this case, the outer case portion 192 may be formed of an insulating material, such as rubber, silicone, or the like. The outer case portion 192 may prevent malfunction of the extracorporeal shock wave medical device 100 which may occur due to externally generated static electricity or the like. In addition, the outer case portion 192 may prevent a high-frequency signal and/or a low-frequency signal generated when the extracorporeal shock wave medical device is operated from being transmitted to the outside of the body portion 130.

The cap portion 193 may be coupled to the outer case portion 192. In this case, the cap portion 193 may include an elastic material, such as rubber, silicone, or the like, or may include a synthetic resin material. A hole may be formed in the cap portion 193 so that a part of the impact portion 160 is exposed to the outside. In another embodiment, the cap portion 193 may further include a contact portion that is in contact with or coupled to the impact portion 160 and is in contact with the user's body. In this case, the contact portion may include a metal material.

Meanwhile, the extracorporeal shock wave medical device 100 as described above may implement various functions. For example, the extracorporeal shock wave medical device 100 may be operated according to a user's signal. More specifically, the user may input a control signal through the handle portion 110 or an input portion 111 disposed outside the handle portion 110. In this case, the control signal ma be a signal to transmit a physical shock to the user or to transmit a high-frequency signal and/or a low-frequency signal to the user. Here, the input portion 111 may be formed in various forms. For example, in one embodiment, the input portion 111 may be formed in the form of button, and may generate different control signals according to a pressing force on the button. In another embodiment, the input portion 111 may generate different control signals according to rotation in the form of a jog shuttle. In still another embodiment, the input portion 111 may be formed in the form of a touch screen. In this case, the input portion 111 is not limited to the above, and may include any form capable of inputting various control signals.

When physical shock is transmitted to the user, a pump may be operated to supply gas to the cylinder 141. In this case, the gas is introduced between the moving portion 142 and the end of the cylinder 141 so that the pressure between the moving portion 142 and the end of the cylinder 141 may be increased. At this time, the magnet portion 122 and/or the core portion 121 may completely shields the end of the cylinder 141 to prevent the gas from flowing out through the end of the cylinder 141. In this case, a separate cover 143 may be disposed at the end of the cylinder 141 to shield the end of the cylinder 141.

In the above case, the magnet portion 122 may impede the movement of the moving portion 142 to some extent, so that the moving portion 142 may not move until the pressure inside the space between the moving portion 142 and the end of the cylinder 141 reaches a predetermined pressure. In this case, the moving portion 142 may include a magnetic material so that the moving portion 142 may not move by the magnetic force of the magnet portion 122. Afterwards, when the pressure inside the space between the moving portion 142 and the end of the cylinder 141 is greater than or equal to the predetermined pressure, the force pushing the moving portion 142 by the pressure may become greater than the magnetic force exerted by the magnet portion 122 on the moving portion 142.

When the moving portion 142 moves, the moving portion 142 may linearly move inside the cylinder 141 along the longitudinal direction of the cylinder 141. Thereafter, the moving portion 142 may collide with the second protruding portion 152b of the second connecting portion 152, and the second protruding portion 152b may pressurize the inserted portion 161 so that the impact transfer portion 162 can advance.

In this case, the pump supplies gas and/or then sucks the gas inside the cylinder 141 to move the moving portion 142 forward or backward along the cylinder 141.

When the moving portion 142 reciprocates as described above, the moving portion 142 may hit the second connecting portion 152 a plurality of times, such as at predetermined time intervals, and accordingly, the impact transfer portion 162 may vibrate. Such vibration may be transmitted to the user's body through the impact transfer portion 162. At this time, the cap portion 193 may change in shape according to the vibration.

In the above case, the user may transmit a high-frequency signal and/or a low-frequency signal to the user's body through the input portion. For example, when the waveform transmitting portion 191 transmits a high-frequency signal to the core portion 121, the high-frequency signal may be sequentially transmitted to the core portion 121, the cylinder 141, the second connecting portion 152, and the impact portion 160 and may reach the user's body.

In the above case, as the moving portion 142 reciprocates the cylinder 141 as described above, the impact portion 160 may vibrate. In this case, as the moving portion 142 hits the second connecting portion 152, the second connecting portion 152 and the end of the cylinder 141 may be momentarily spaced apart from each other, or the second connecting portion 152 and the impact portion 160 may be spaced apart from each other. At this time, a certain gap may be generated between the second connecting portion 152 and the end of the cylinder 141 and/or between the second connecting portion 152 and the impact portion 160, and such a gap may serve as a capacitor momentarily. However, the momentarily generated capacitor may undergo an overcurrent situation due to the concentration of excessive charges and provide excessive energy to the user through the impact portion 160, thereby damaging the user's body or causing discomfort to the user.

However, in this case, the contact holding portion 170 may prevent such a gap from being generated. That is, the contact holding portion 170 may electrically connect between the second connecting portion 152 and the end of the cylinder 141 and/or between the second connecting portion 152 and the impact portion 160 at all times as described above, so that the problem as described above may be resolved.

On the other hand, the problem as described above may occur even when the waveform transmitting portion 191 generates and transmits a low-frequency signal. In addition, the same problem may apply to the case where the waveform transmitting portion 191 mixes the high-frequency signal and the low-frequency signal and provides the mixed signals to the impact portion 160.

In the case where the moving portion 142 is moved as described above, the moving portion 142 may be moved backward inside the cylinder 141 as the pump discharges the air inside the cylinder 141 to the outside and the magnet portion 122 provides a magnetic force to the moving portion 142. In addition, the magnet portion 122 may fix the position of the moving portion 142 or move the moving portion 142 backward by applying a magnetic force to the moving portion 142 when the moving portion 142 reaches a predetermined position inside the cylinder 141.

Accordingly, the extracorporeal shock wave medical device 100 may prevent damage to the user's body due to the high-frequency signal and/or the low-frequency signal even when the high-frequency signal and/or the low-frequency signal are provided to the user and the vibration is applied at the same time.

In addition, the extracorporeal shock wave medical device 100 may provide the user with a high-frequency signal and/or a low-frequency signal having a certain magnitude.

FIG. 5 is a cross-sectional view showing a connecting portion, an impact portion, and a contact holding portion of an extracorporeal shock wave medical device according to another embodiment of the present invention.

Referring to FIG. 5, an extracorporeal shock wave medical device (not shown) may include a handle portion (not shown), a core portion (not shown), a magnet portion (not shown), a body portion (not shown), a cylinder 141, a moving portion (not shown), a first connecting portion (not shown), a second connecting portion 152, an impact portion 160, a waveform transmitting portion (not shown), a contact holding portion 170, a sealing portion (not shown), an outer case portion (not shown), a cap portion (not shown), and a cover (not shown). In this case, the handle portion, the core portion, the magnet portion, the body portion, the cylinder 141, the moving portion, the first connecting portion, the second connecting portion 152, the waveform transmitting portion, the sealing portion, the outer case portion, the cap portion, and the cover may be the same as or similar to those described with reference to FIGS. 1 to 4, and thus detailed descriptions thereof will be omitted.

The impact portion 160 may include an inserted portion 161 and an impact transfer portion 162. In this case, the impact transfer portion 162 may be provided with a position limiting portion 162b protruding from the outer surface of the impact transfer portion 162 to limit the position of a first contact holding portion 171 which will be described below. In this case, the position limiting portion 162b may be disposed inside the first contact holding portion 171.

The contact holding portion 170 may include the first contact holding portion 171 and a second contact holding portion 172. In this case, the first contact holding portion 171 may include a spring and may be disposed between the impact transfer portion 162 and the second connecting portion 152. The second contact holding portion 172 may include a magnet which is disposed on the magnet portion 161 and/or on the second connecting portion 152. The magnet may be in the form of a permanent magnet and may prevent a second protruding portion 152b and the inserted portion 161 from separating from each other when the moving portion hits the second protruding portion 152b.

Accordingly, the extracorporeal shock wave medical device may stably provide a high-frequency signal and/or a low-frequency signal while transferring physical vibrations to a user.

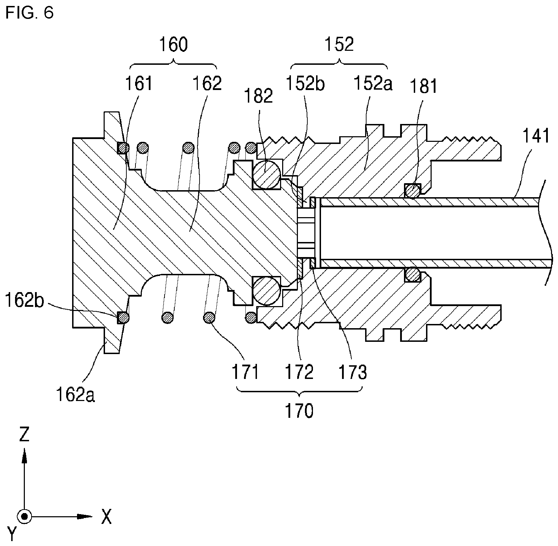

FIG. 6 is a cross-sectional view showing a connecting portion, an impact portion, and a contact holding portion of an extracorporeal shock wave medical device according to still another embodiment of the present invention.

Referring to FIG. 6, an extracorporeal shock wave medical device (not shown) may include a handle portion (not shown), a core portion (not shown), a magnet portion (not shown), a body portion (not shown), a cylinder 141, a moving portion (not shown), a first connecting portion (not shown), a second connecting portion 152, an impact portion 160, a waveform transmitting portion (not shown), a contact holding portion 170, a sealing portion (not shown), an outer case portion (not shown), a cap portion (not shown), and a cover (not shown). In this case, the handle portion, the core portion, the magnet portion, the body portion, the cylinder 141, the moving portion, the first connecting portion, the second connecting portion 152, the waveform transmitting portion, the sealing portion, the outer case portion, the cap portion, and the cover may be the same as or similar to those described with reference to FIGS. 1 to 4, and thus detailed descriptions thereof will be omitted.

The impact portion 160 may include an inserted portion 161 and an impact transfer portion 162. In this case, the impact transfer portion 162 may be provided with a position limiting portion 162b in the form of a groove recessed from the outer surface of the impact transfer portion 152 to limit the position of a first contact holding portion 171 which will be described below.

The contact holding portion 170 may include the first contact holding portion 171, a second contact holding portion 172, and a third contact holding portion 173. In this case, the first contact holding portion 171 may include a spring and may be disposed between the impact transfer portion 162 and the second connecting portion 152. In addition, the second contact holding portion 172 may include a magnet which is disposed on the magnet portion 161 and/or on the second connecting portion 152. The magnet may be in the form of a permanent magnet and may prevent a second protruding portion 152b and the inserted portion 161 from separating from each other when the moving portion hits the second protruding portion 152b. In addition, the third contact holding portion 173 may include a magnet that is disposed on the cylinder 141 and/or on the second connecting portion 152. The magnet may be in the form of a permanent magnet and may prevent the second protruding portion 152b and the end of the cylinder 141 from separating from each other when the second protruding portion 152b hits the moving portion.

Accordingly, the extracorporeal shock wave medical device may stably provide a high-frequency signal and/or a low-frequency signal while transferring physical vibrations to a user.

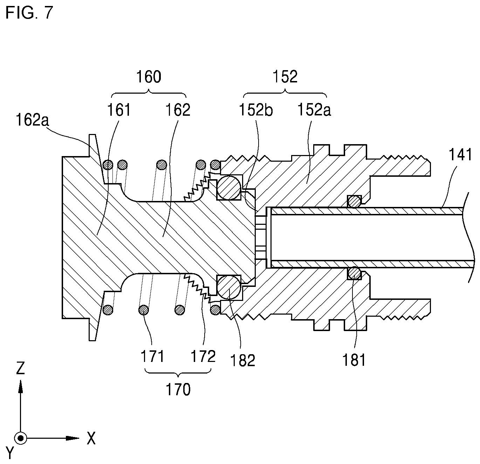

FIG. 7 is a cross-sectional view showing a connecting portion, an impact portion, and a contact holding portion of an extracorporeal shock wave medical device according to another embodiment of the present invention.

Referring to FIG. 7, an extracorporeal shock wave medical device (not shown) may include a handle portion (not shown), a core portion (not shown), a magnet portion (not shown), a body portion (not shown), a cylinder 141, a moving portion (not shown), a first connecting portion (not shown), a second connecting portion 152, an impact portion 160, a waveform transmitting portion (not shown), a contact holding portion 170, a sealing portion (not shown), an outer case portion (not shown), a cap portion (not shown), and a cover (not shown). In this case, the handle portion, the core portion, the magnet portion, the body portion, the cylinder 141, the moving portion, the first connecting portion, the second connecting portion 152, the impact portion 160, the waveform transmitting portion, the sealing portion, the outer case portion, the cap portion, and the cover may be the same as or similar to those described with reference to FIGS. 1 to 4, and thus detailed descriptions thereof will be omitted.

The impact portion 160 may include an inserted portion 161 and an impact transfer portion 162. In this case, as illustrated in FIGS. 6 and 7, the impact transfer portion 162 may be provided with a position limiting portion in the form of a groove recessed from the outer surface of the impact transfer portion 162 or in the form of a protrusion to limit the position of a first contact holding portion 171 which will be described below.

The contact holding portion 170 may include the first contact holding portion 171 and a second contact holding portion 172. In this case, the first contact holding portion 171 may include a spring and may be disposed between the impact transfer portion 162 and the second connecting portion 152. In addition, the second contact holding portion 172 may include a wire disposed between the impact portion 160 and the second connecting portion 152 to electrically connect the impact portion 160 and the second connecting portion 152. At this time, one end of the second contact holding portion 172 may be attached or fixed to various positions of the impact portion 160 and the other end of the second contact holding portion 172 may be attached or fixed to the second connecting portion 152. In this case, the ends of the second contact holding portion 172 may be inserted and fixed into the outer surface of the impact portion 160 and the surface of the second connecting portion 152, respectively. In another embodiment, each end of the second contact holding portion 172 may be fixed to the outer surface of the impact portion 150 and the surface of the second connecting portion 152 by welding. In still another embodiment, each end of the second contact holding portion 172 may include a magnet which is attached and fixed to the outer surface of the impact portion 160 and the surface of the second connecting portion 152. In yet another embodiment, each end of the second contact holding portion 172 may be fixed to the outer surface of the impact portion 160 and the surface of the second connecting portion 152 with a tape or the like. In such a case, the impact portion 160 and the second connecting portion 152 may not only be electrically connected to each other, but also may be prevented from separating from each other when the moving portion hits the second protruding portion 152b.

Accordingly, the extracorporeal shock wave medical device may stably provide a high-frequency signal and/or a low-frequency signal while transferring physical vibrations to a user.

While one or more example embodiments have been described with reference to the figures, it will be understood by those of ordinary skill in the art that various changes in form and details may be made therein without departing from the spirit and scope as defined by the following claims.

### INDUSTRIAL APPLICABILITY

According to an embodiment of the present invention, an extracorporeal shock wave medical device that simultaneously provides mechanical vibration and electrical stimulation is provided, and embodiments of the present invention can be efficiently applied to a medical device that stimulates various lesions, a medical device and a cosmetic device for preventing skin aging through skin stimulation, and the like.

## Claims

1. An extracorporeal shock wave medical device comprising:
a body portion;
a cylinder disposed inside the body portion;
a moving portion which linearly moves inside the cylinder;
a connecting portion disposed at an end of the cylinder and connected to the body portion to selectively collide with the moving portion when the moving portion moves;
an impact portion connected to the connecting portion and configured to transmit an impact to the outside when the moving portion collides with the connecting portion; and
a waveform transmitting portion electrically connected to the impact portion to transmit a high frequency or low frequency to the impact portion.

2. The extracorporeal shock wave medical device of claim 1, further comprising a cap portion connected to the impact to transmit the high frequency or low frequency to a user.

3. The extracorporeal shock wave medical device of claim 1, further comprising an outer case portion disposed to surround the outside of the body portion.

4. The extracorporeal shock wave medical device of claim 1, further comprising a contact holding portion disposed between the connecting portion and the impact portion to electrically connect the connecting portion and the impact portion.

5. The extracorporeal shock wave medical device of claim 4, wherein the contact holding portion comprises a spring which is a conductive material.

6. The extracorporeal shock wave medical device of claim 1, wherein the connecting portion comprises a connecting body portion into which a part of the impact portion is inserted, and a protruding portion which protrudes to the inside of the connecting body portion and is in contact with the part of the impact portion.
